# EUROPEAN PATENT SPECIFICATION

(11) **EP 4 018 372 B1**
(45) Date of publication and mention of the grant of the patent: **02.10.2024**
(21) Application number: 20856416.1
(22) Date of filing: 25.08.2020
(51) Int. Cl.: A01K 67/033, A01M 1/02, A01K 29/00, G06N 5/02, B07C 5/342, B07C 5/36, A01M 1/22, G06N 3/08, G06V 10/141, G06V 20/00, G06V 20/66

(54) **SYSTEM AND METHOD FOR SEX-SORTING OF PRE-ADULT INSECTS**
SYSTEM UND VERFAHREN ZUR GESCHLECHTSSORTIERUNG VON PRÄADULTEN INSEKTEN
SYSTÈME ET PROCÉDÉ DE TRI PAR SEXE D'INSECTES PRÉ-ADULTES

(30) Priority: 25.08.2019 US 201962891360 P
(43) Date of publication of application: 29.06.2022
(73) Proprietor: Diptera.AI Ltd., 9139001 Jerusalem (IL)
(72) Inventor: LIVNE, Ariel, 9311514 Jerusalem (IL); ORDAN, Elly, 9387420 Jerusalem (IL)
(74) Representative: Schulz Junghans Patentanwälte PartGmbB
(86) International application number: PCT/IL2020/050927
(87) International publication number: WO 2021/038561

(56) References cited:
- EP-A1- 3 430 900
- WO-A1-2017/158216
- WO-A1-2019/045648
- WO-A2-2018/134828
- WO-A2-2019/008591
- WO-A2-2019/008591
- CN-A- 107 838 054
- US-A1- 2014 226 860

## Description

### FIELD OF THE INVENTION

The present invention relates to the field of sorting insects. More specifically, the present invention relates to a system and method for sex-sorting of pre adult insects.

### BACKGROUND OF THE INVENTION

The identification and separation of male and female insects is necessary in various agricultural and industrial aspects. For example, industrial insect rearing, e.g. as a source of protein or for waste disposal, seeks to optimize reproduction for increased yield. This depends, in part, on the sex-ratio of males-females in the breeding cages. In another example, insects play an important role in genetic research, as they have short life cycles and are simple to work with and contain. The identification and separation of male and female insects are required in any genetic study. Large scale sex separation is further considered highly desirable or essential for the sterile insect technique (SIT). SIT is an insect pest control method involving the mass-rearing and sterilization, e.g. using radiation, genetic manipulation, chemical sterilization, RNAi and Wolbachia infection, of a target pest, followed by the systematic area-wide release of the sterile males, over defined areas. The sterile males mate with wild females resulting in fewer offspring and a declining pest population. The International Plant Protection Convention categorizes sterile insects as beneficial organisms. SIT differs from classical biological control, which involves the introduction of non-native biological control agents, in several ways: (1) sterile insects are not self -replicating and therefore cannot become established in the environment, (2) breaking the pest's reproductive cycle, also called autocidal control, is by definition species-specific, and (3) the SIT does not introduce non-native species into an ecosystem.

The application of the sterile insect technique (SIT) requires an efficient and effective sex-separation system. Sex-separation systems eliminate females from the production line and prior to field release of sterile males. This is particularly important for mosquito control because females bite and can transmit diseases and their release, therefore, must be prevented.

Current, biological sex separation methods have drawbacks such as relying on sexually dimorphic characters (e.g., size or development rate) that are subject to natural variation, requiring regular adjustment and re-calibration of the sorting systems used. They are performed at the pupae and/or adult stage.

Sex-separation methods based on the use and release of genetically modified organisms encounter regulation and public opposition.

Several solutions attempting to automate the separation for adult mosquitoes, are described in the art.

PCT application WO/2018/134829 discloses an apparatus and method for mechanical sex-sorting of mosquitoes by extracting a class of mosquitoes from unsorted mosquitoes. This publication teaches separation of the mosquitoes using a robotic arm.

US Patent Application 2018121764 discloses a computer-implemented method and system for classifying insects according to predetermined categories. The aforementioned US application teaches that the mosquitoes are analyzed when they are on a surface.

US Patent 10278368 discloses an insect separating apparatus. The disclosed separation method is based on information indicative of a Doppler shift caused by movement of the insect. It is taught by this patent to classify mosquitoes mainly by their wingbeat frequency (using Doppler). It is therefore designed for flying insects and not larvae or pupae.

US Patent Application 2018092339 discloses an automated insect rearing system comprising: an insect dispensing station, a food dispensing station and a robotic storage system configured to move the rearing container. This US application further discloses that the rearing system may comprise an automated sex sorting unit that includes an imaging device and an ultrasound for sorting adult insects that have matured from the larvae within the rearing container. It is taught that pupae can be sorted by features such as size, weight, etc. In addition, a two stage approach is suggested, before the pupae emerge into adults, an imaging device is used to sort the pupae based on their size. After the pupae emerge into adults the sex sorting is done by wingbeat frequency.

US Patent 10178857 discloses a system that is usable to separate a group of insects using non-forcible means to analyze the characteristics of the insects and sort them based on these characteristics. The system includes a singulation chamber having specified features (ramped surfaces) that encourage mosquitoes to voluntarily navigate through the singulation chamber in a single-file manner to be sorted based on their physical characteristics (e.g., shape, size, etc.). Thus, this patent teaches analyzing insects that reach the test region in a single-file. The insects should be adult mosquitoes that either fly or walk.
WO 2019/008591 presents a method for sorting insects into classes or for quality control of sorted insects. The method involves placing the insects in a container with a closable opening, then releasing the insects through this opening into a funneled air flow. The air flow directs the insects to a conveyable collection location. Upon reaching this location, an immobilizing agent is applied to the insects, rendering them stationary for subsequent sorting or quality control purposes. This approach emphasizes efficient handling and preparation of insects for detailed analysis or classification based on predetermined criteria

CN107838054 A discloses a biological sorting device for the detection and sorting of organisms such as nematodes.

In view of the above, there is still a long felt and unmet need for an efficient and cost-effective technique for rapid sex-sorting of pre- adult insects.

### SUMMARY OF THE INVENTION

Accordingly, it is a principal object of the present invention to overcome disadvantages of prior art. This is accomplished in one embodiment by disclosing a system for sex separation of pre-adult insects comprising: (a) fluidic channel having an inner space and an outer wall, said fluidic channel comprising an inlet and at least one outlet, each one of said one or more outlets is associated with a classification category selected from the group consisting of male insect, female insect and other, wherein said channel is configured to allow flow of pre-adult insects suspended in liquid media; (b) a processor; and, (c) a controller in-communication with said processor; wherein said fluidic channel comprises a classification region, and the classification region comprises an electro-optical module in-communication with the processor, said electro-optical module comprises at least one sensor configured to acquire optical data of an individual pre-adult insect while suspended in liquid media and flowing in said fluidic channel, and to transmit said optical data to the processor, further wherein the processor is configured to process the acquired optical data, to classify said pre-adult insect based on the acquired optical data, and to instruct the controller to sort said pre-adult insect based on the classification of said pre-adult insect.

It is a further object of the present invention to disclose the system as defined in any of the above, wherein said fluidic channel comprising more than one outlet, and wherein each outlet is independently operated by the controller; the configuration of the outlet switches from 'open' to 'closed', and from 'closed to 'open'.

It is a further object of the present invention to disclose the system as defined in any of the above, wherein the fluidic channel further comprises a separation region, wherein said separation region is located between the classification region and the plurality of outlets; wherein said separation region comprises at least one insect-guiding tool configured to guide said pre-adult insect to an outlet associated with the insect classification, and wherein the insect-guiding tool is selected from the group consisting of one or more valves, a transducer configured to generate acoustic radiation forces, electrodes configured to apply electroosmotic forces to the liquid media, electrodes configured to apply dielectrophoretic forces to the pre-adult insect and any combination thereof.

It is a further object of the present invention to disclose the system as defined in any of the above, wherein the fluidic channel further comprises a destruction region; wherein said destruction region comprises at least one insect- destroying tool in-communication with the controller; and wherein said insect- destroying region has an "on" and "off' configurations operated by the controller.

It is a further object of the present invention to disclose the system as defined in any of the above, wherein the fluidic channel further comprises a destruction region; wherein said destruction region comprises at least one insect- destroying tool in-communication with the controller; and wherein said insect- destroying region has an "on" and "off' configurations operated by the controller.

It is a further object of the present invention to disclose the system as defined in any of the above, wherein the electro-optical module of the classification region comprises at least one of: one or more light sources configured to illuminate the classification region by emitting light with predetermined spectrum and/or intensities, one or more image sensors, one or more acoustic sources configured to generate sound waves that pass through the classification region, at least one optical element and an internal control unit in-communication with the processor, wherein the electro-optical module is configured to obtain multiple images of the pre-adult insect from different angles, and wherein the light source illumination is stroboscopic or pulsed.

It is a further object of the present invention to disclose the system as defined in any of the above, wherein said system further comprising a sensor in communication with the controller, wherein the controller is further configured to control the amount of insect individuals at the entrance to the fluidic channel based on the data acquired by said sensor, to thereby allow passage of single pre-adult insect in a time to the fluidic channel.

It is a further object of the present invention to disclose the system as defined in any of the above, wherein the processor is configured to perform functions selected from receiving the optical data of an individual pre-adult insect acquired by the sensor of the electro-optical module, processing the optical data, extracting a set of parameters indicative of the sex of the pre-adult insect from the optical data, classifying the pre-adult insect based on the set of parameters extracted from the optical data, providing instructions to the controller based on the classification of the pre adult insect.

It is a further object of the present invention to disclose the system as defined in any of the above, wherein the parameters indicative of the sex of the pre-adult insect are selected from the group consisting of overall size of the pre adult insect, morphology of the pre-adult insect, shape, segment size ratio, IR (Infrared) absorption, color, fluorescence, gonad disc morphology, secondary sex organ morphology, gonad size, gonad morphology, gonad autofluorescence, size of testes, size of male accessory glands, morphology of testes, morphology of male accessory glands, autofluorescence of testes, autofluorescence of male accessory glands, size of the developing male and female primary or secondary reproductive structures, primitive sex organs, morphology of the developing male and female primary or secondary reproductive structures, autofluorescence of the developing male and female primary or secondary reproductive structures and any combination thereof.

It is a further object of the present invention to disclose the system as defined in any of the above, wherein said system further comprises a container in fluid connection with a fluidic channel for sex separation of pre-adult insects, said container contains unsorted pre-adult insects suspended in a liquid media, and having an outlet configured to allow passage of a single pre adult insect at a time to the fluidic channel, said container further comprises pressurizing means, such as pressurized gas, vacuum, gravity forces and/or one or more pumps to drive a flow of unsorted pre-adult insects towards the outlet of the container to the fluidic channel.

It is a further object of the present invention to disclose a computer implemented method of sex separation of pre-adult insects comprising: (a) providing the system for sex separation of pre-adult insects as defined in any of the above; (b) streaming the pre-adult insects suspended in the liquid media through the inlet into the fluidic channel towards the classification region; (c) acquiring optical data of the individual pre-adult insect at the classification region by one or more sensors of the electro-optical module module; (d) transmitting the optical data of the individual pre-adult insect to the processor; (e) processing the optical data and classifying the individual pre-adult insect based on a set of parameters indicative of the sex of the pre-adult insect extracted from the optical data; (f) providing instructions to the controller based on the classification of the individual pre-adult insect; and, (g) sorting the pre-adult insect according to the instructions received by the controller.

It is a further object of the present invention to disclose the method as defined in any of the above, wherein the pre-adult insects are classified into at least two classes selected from a group consisting of: male and non-male, or female and non-female.

It is a further object of the present invention to disclose the method as defined above, wherein the pre-adult insect is a larva or a pupa or a nymph.

It is a further object of the present invention to disclose the method as defined in any of the above, wherein the pre-adult insect type is selected from the group of Endopterygota consisting of: Coleoptera (Beetles), Diptera (Flies), Hymenoptera (Ants, bees, sawflies and wasps), Lepidoptera (Butterflies, and moths), Mecoptera (Scorpionflies), Megaloptera (Alderflies, dobsonflies, and fishflies), Miomoptera, Neuroptera (Lacewings, antiions), Raphidioptera (Snakeflies), Siphonaptera (Fleas), Strepsiptera (Twisted-winged parasites) and Trichoptera (Caddisflies), and wherein the pre-adult insect species is selected from the group consisting of: a) Diptera species, such as from the family Culicidae, e.g. Culex pipiens, Aedes Aegypti, Aedes albopictus, Anopheles gambiae; from the family Drosophilidae, e.g. Drosophila melanogaster, Drosophila suzukir, from the family Tephritidae, e.g. Bactrocera tryoni, Anastrepha fraterculus, Anastrepha ludens; from the family Stratiomyidae, e.g. Hermetia illucens from the family Syrphidae, e.g. Eristalis tenax b) Fepidoptera species, such as from the family Plutellidae, e.g. Plutella xylostella from the family Pyralidae, e.g. Plodia interpunctella c) Coleoptera species, such as from the family Tenebrionidae, e.g. Tribolium castaneum.

It is a further object of the present invention to disclose the method as defined in any of the above, further comprises steps of reducing or elevating the temperature of the liquid media so as to control wriggling movements of the pre-adult insects flowing within the fluidic channel.

It is a further object of the present invention to disclose the method as defined in any of the above, wherein said classifying comprises using a trained neural network.

### BRIEF DESCRIPTION OF THE DRAWINGS

For a better understanding of the invention and to show how the same may be carried into effect, reference will now be made, purely by way of example, to the accompanying drawings in which like numerals designate corresponding elements or sections throughout.

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. In the accompanying drawings:
Fig. 1 is an illustrative flow chart of embodiments of the present invention describing a flow system to classify larvae and/or pupae and sorting them accordingly;
Figs 2A-2C schematically illustrate non-limiting examples of a flow system with integrated classification region and sorting region (Figs 2A-B) and optionally destruction region (Fig. 2C), according to some embodiments of the present invention; and
Fig. 3 schematically illustrates a container with unsorted larvae and/or pupae, in accordance with certain embodiments of the present invention.

### DETAILED DESCRIPTION OF THE OF THE INVENTION

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

The present invention provides a novel, rapid and cost effective technique of sex-sorting of larvae and/or pupae and/or nymph developmental stages. The process as inter-alia disclosed is automated and preferably does not require user intervention. In some of the aspects, image analysis algorithms employed in the herein disclosed process and system to ensure an accurate separation of the insects. Furthermore, the separation is indifferent to the type of mosquito (or other insects) and how it is sterilized. These advantages enable to use the system and method of the present invention for commercial rearing applications for mosquitoes (or other insects).

Sex-separation at pre-adult stages, e.g. larval or pupal stage, has a number of advantages over separation at the adult stage. Firstly, the females are disposed of (i.e. removed from the rearing population) at an earlier stage thus saving food, space and energy in the rearing process. Secondly, since the sterile males have a limited life span (typically about 10 days for mosquitoes), separation at an early developmental stage, facilitates their release at an earlier stage (e.g., shortly after emerging from the pupal case), thus increasing their exposure to females. Thirdly,
separation at the larval or pupal stage provides more time for transporting the male insects from the rearing facility to the target site of release. In addition, the transport itself may also be simpler since larvae and/or pupae are less fragile than adult insects.

The invention provides, in some of its aspects, a novel fluidic channel comprising an inlet and outlet(s); a classification region or unit or zone or module and a separation region or unit or zone or module and/or a destruction region or unit or zone or module. The channel is configured to receive larvae and/or pupae suspended in a liquid. The larvae and/or pupae pass through a classification region where they are imaged and classified according to their sex. According to this classification, in some embodiments, the larvae and/or pupae are separated into different outlets, where they can then be collected. In some embodiments, the larvae and/or pupae are classified according to their sex with one class passing undamaged to the single outlet for collection, and the rest being destroyed.

The present invention provides a system for sex separation of pre-adult insects comprising: (a) a fluidic channel having an inner space and an outer wall, said fluidic channel comprising an inlet and at least one outlet, wherein said channel is configured to allow flow of pre-adult insects suspended in liquid media; (b) a processor; and, (c) a controller in-communication with said processor. In core aspects of the present invention, the fluidic channel comprises a classification region, and the classification region comprises an electro-optical module in-communication with the processor, said electro-optical module comprises at least one sensor configured to acquire optical data of an individual pre-adult insect and to transmit said data to the processor, further wherein the processor is configured to process the acquired data, to classify said pre-adult insect based on the acquired data, and to instruct the controller to sort said pre-adult insect based on the classification of said pre-adult insect.

The present invention further provides a computer implemented method of sex separation of pre-adult insects comprising steps of: (a) providing the system for sex separation of pre-adult insects as defined in any of the above; (b) streaming the pre adult insects suspended in the liquid media through the inlet into the fluidic channel towards the classification region; (c) acquiring optical data of the individual pre-adult insect at the classification region by one or more sensors of the electro-optical module; (d) transmitting the optical data of the individual pre-adult insect to the processor; (e) processing the optical data and classifying the individual pre-adult insect based on a set of parameters indicative of the sex of the pre-adult insect extracted from the data; (f) providing instructions to the controller based on the classification of the individual pre adult insect; and, (g) sorting the pre-adult insect according to the instructions received by the controller. In other main aspects, the present invention provides a computer implemented algorithm comprising code to perform the steps of the method as defined in any of the above.

As used herein, the terms "comprises", "comprising", "includes",
"including", "having" and their conjugates mean "including but not limited to".

As used herein, the singular form "a", "an" and "the" include plural references unless the context clearly dictates otherwise. For example, the term "a compound" or "at least one compound" may include a plurality of compounds, including mixtures thereof.

The use of "adapted to" or "configured to" herein is meant as open and inclusive language that does not foreclose devices adapted to or configured to perform additional tasks or steps. Additionally, the use of "based on" is meant to be open and inclusive, in that a process, step, calculation, or other action "based on" one or more recited conditions or values may, in practice, be based on additional conditions or values beyond those recited. Similarly, the use of "based at least in part on" is meant to be open and inclusive, in that a process, step, calculation, or other action "based at least in part on" one or more recited conditions or values may, in practice, be based on additional conditions or values beyond those recited. Headings, lists, and numbering included herein are for ease of explanation only and are not meant to be limiting.

The term "about" as used herein denotes ± 25% of the defined amount or measure or value.

The term "insect" as used herein generally refers to hexapod invertebrates class within the arthropod phylum. Insects have segmented bodies, jointed legs, and external skeletons (exoskeletons). According to further aspects, an arthropod in the class Insecta is characterized by six legs, up to four wings, and a chitinous exo skeleton.

The term "Endopterygota" as used herein, also refers to as as Holometabola, is understood to relate to a superorder of insects within the infraclass Neoptera that go through distinctive larval, pupal, and adult stages. They undergo a radical metamorphosis, with the larval and adult stages differing considerably in their structure and behavior. This process is also called holometabolism, or complete metamorphism. In certain aspects of the present invention, the Endopterygota constitute the most diverse insect superorder, with about 11 orders, containing insects such as butterflies, flies, fleas, bees, ants, and beetles.

Examples of insect types within the scope of the present invention include, orders such as: Coleoptera - Beetles, Diptera - Flies, Hymenoptera - Ants, bees, sawflies, and wasps, Lepidoptera - Butterflies, and moths, Mecoptera -Scorpionflies, Megaloptera - Alderflies, dobsonflies, and fishflies, Miomoptera (extinct), Neuroptera - Lacewings, antiions, etc., Raphidioptera - Snakeflies, Siphonaptera - Fleas, Strepsiptera - Twisted-winged parasites and Trichoptera -Caddisflies.

More particularly, insect types within the scope of the present invention include, but are not limited to mosquito species, e.g. of the genus: Culex, Aedes, Anopheles, Bactrocera, Hermetia, Anastrepha and Eristalis, Drosophilidae species, e.g. of the genus: Drosophila, moth species, for example of the family Plutellidae and/or Pyralidae, e.g. of the genus Plutella, fly species, for example of the family Tephritidae and/or Stratiomyidae,, beetle species, for example of the family Tenebrionidae, e.g. the genus Tribolium, and Lepidoptera, for example of the genus: Plodia.

Even more specific non-limiting examples of insect species within the scope of the present invention include Culex pipiens, Aedes Aegypti, Aedes albopictus, Anopheles gambiae, Drosophila melanogaster, Plutella xylostella, Bactrocera tryoni, Drosophila suzukii, Hermetia illucens, Anastrepha fraterculus, Anastrepha ludens, Tribolium castaneum, Plodia interpunctella and Eristalis tenax.

The term "pre-adult" as used herein refers in the context of the present invention to stages of an insect life cycle including larval, pupal, nymph or any other juvenile stages. In some aspects it refers to non-adult stage, imaginal stage, immature instar developmental stages, immature or nymph stage.

The term "fluidic channel" as used herein refers to a device, apparatus or a container having an inner space and outer walls, wherein the inner space is contained with and/or allow the passage of a flowable fluid or liquid media or solution, for example water or isotonic aqueous solution. Such a fluidic channel has at least one inlet and at least one outlet and in the context of the present invention is adapted to contain pre-adult insects suspended in a liquid media passing or flowing from the inlet portion of the channel towards the outlet potion of the channel while subjected to automatic/computed classification process from unsorted insect to sex-sorted insect (e.g. male or female insect).

The term "movement" in the context of the invention is meant to be understood as voluntary or un-voluntary movement of the pre-adult insect as it passes through the sorting system.

The term "streaming" herein refers to flowing or rapidly flowing of a liquid or fluid or suspension, e.g. a velocity or volume flow in a channel.

The term "liquid media" refers, without limitation, to a substance in a physical state in which it does not resist change of shape but does resist change of size.

The term "passive flow" refers to flow control methods which require no auxiliary power. The non-limiting list of passive flow control methods includes any passive devices which are steady and require no energy by definition (e.g. derived by gravity forces), such as turbulators, roughness elements or any other passive device influencing the fluid dynamics of the flow.

The term "active flow" refers to flow control methods which require energy expenditure. The non-limiting list of active flow control methods includes valves, pumps, pressurizing means, applying a positive or negative (vacuum) pressure difference with respect to parts of the system of the present invention or any other artificially driven actuator which require energy.

It is within the scope of the present invention that the system and method for sex-separation or sorting of pre-adult insect comprises means to drive the insects out of a container containing unsorted insects and through the system that may be connected to such a container towards the outlet of such a system. These include vacuum, valves, gravity forces and pumps. These means may further include insect-guiding tool configured to guide the pre-adult insect to an outlet associated with the insect classification. Examples of such an insect-guiding tool may comprise one or more valves, a transducer configured to generate acoustic radiation forces, electrodes configured to apply electroosmotic forces to the liquid media, electrodes configured to apply dielectrophoretic forces to the pre-adult insect and any combination thereof.

The term "controller" refers, without limitation, to any hardware device or a software program, or a combination of the two that manages or directs the flow of data between two or more entities. In a general sense, a controller can be thought of as something or someone that interfaces between two systems and manages communications between them.

The system of the present invention may comprise a control center, wherein said control center is configured to monitor system performance.

It is within the scope of the present invention that system, e.g. using the classification region, processor and controller parts is configured to classify an individual pre-adult insect into classes selected from the group consisting of male, female, and/or other by a set of parameters indicative of the sex of the pre-adult insect (e.g. based on optical and/or other data of the insect).

As used herein, the term "parameters indicative of the sex of the pre adult insect" includes, in a non-limiting manner: overall size of the pre-adult insect, morphology of the pre-adult insect, shape, segment size ratio, IR (Infrared) absorption, color, fluorescence, gonad disc morphology, secondary sex organ morphology, gonad size, gonad morphology, gonad autofluorescence, size of testes, size of male accessory glands, morphology of testes, morphology of male accessory glands, autofluorescence of testes, autofluorescence of male accessory glands, size of the developing male and female primary or secondary reproductive structures, primitive sex organs, morphology of the developing male and female primary or secondary reproductive structures, autofluorescence of the developing male and female primary or secondary reproductive structures and any combination thereof.

The term "Stroboscopic illumination system" refers to a system capable of emitting short pulses of light (1 micro second- 10 milliseconds). The pulses of lights can be generated by: light emitting diodes (LEDs), lasers, solid state light sources and Xenon lamps. This system minimizes illumination time, thus reducing phototoxicity and photobleaching artifacts as well as blurring due to motion.

The term "High Frame Rate Camera" refers to a camera capable of capturing images with exposures of less than 1/1,000 second or frame rates in excess of 30 frames per second. It might be useful for recording fast-moving objects as photographic images onto a storage medium.

The term "system performance parameters" refers to key system capabilities that must be met in order for a system to meet its operational goals. A non limiting list of system performance parameters of the invention includes: flow rate, sorting rate, sorting accuracy, insect count, specificity, sensitivity, survival, fitness and throughput.

The terms "accuracy", "specificity" and "sensitivity" of a test may be defined by in terms of TP, TN, FN and FP as detailed below. In the context of the present invention, the tested parameter may be male or female insect, in a particular example, male insect).

True positive (TP) = the number of cases correctly identified as positive for the tested parameter.

False positive (FP) = the number of cases incorrectly identified as positive for the tested parameter.

True negative (TN) = the number of cases correctly identified as negative for the tested parameter.

False negative (FN) = the number of cases incorrectly identified as negative for the tested parameter.

"Accuracy": The accuracy of a test is its ability to differentiate the positive and negative cases correctly. To estimate the accuracy of a test, the proportion of true positive (e.g. male insects) and true negative (e.g. female insects) in all evaluated cases should be calculated. Mathematically, this can be stated as: Accuracy = (TN+TP)/(TN+TP+FN+FP) = (Number of correct assessments)/Number of all assessments)

Sensitivity: The sensitivity of a test is its ability to determine the cases positive for the tested parameter (e.g. male insects) correctly. To estimate it, the proportion of true positive in cases evaluated positive for the tested parameter (e.g. male insects) should be calculated. Mathematically, this can be stated as: Sensitivity = TP/(TP+FN) = (Number of true positive assessment)/(Number of all positive assessment)

Specificity: The specificity of a test is its ability to determine the cases negative for the tested parameter (e.g. female insects) correctly. To estimate it, the proportion of true negative in cases evaluated negative for the tested parameter (e.g. female insects) should be calculated. Mathematically, this can be stated as: Specificity = TN/(TN+FP) = (Number of true negative assessment)/(Number of all negative assessment)

It is herein acknowledged that morphology is a branch of biology dealing with the study of the form and structure of organisms and their specific structural features. This includes aspects of the outward appearance (shape, structure, colour, pattern, size), i.e. external morphology (or eidonomy), as well as the form and structure of the internal parts like bones and organs, i.e. internal morphology (or anatomy). Morphology is a branch of life science dealing with the study of gross structure of an organism or taxon and its component parts.

The term "electro-optical module" as used herein refers to a module or unit or region comprising devices (e.g. Lasers, LEDs, waveguides etc.) and/or systems and/or sensors which operate by the propagation and interaction of light (electromagnetic or optical) and the electrical (electronic) state. Electro-optical sensor is an electronic detector that convert light, or a change in light, into an electronic signal. These sensors are able to detect electromagnetic radiation from the infrared up to the ultraviolet wavelengths. An optical sensor converts light rays into electronic signals. Without wishing to be bound by theory, the electro-optic effect is a change in the optical properties due to interaction with light. Such a module may serve the functions of light modulation, signal routing, tunable filtering, and polarization conversion. They have potential applications such as wide-band optical communication, optical signal processing, and sensors. In the context of the present invention, the electro-optical module of the classification region comprises at least one of: one or more light sources configured to illuminate the classification region, one or more acoustic sources configured to generate sound waves that pass through the classification region, one or more image sensors, at least one optical element and an internal control unit in communication with the processor.

According to one embodiment, the electro -optical module comprises more than one light source. In certain aspects, each light source is configured to emit light with predetermined spectrum and/or intensities. More particularly, illumination may be broadband (for example, white light) or monochromatic.

The term "sensor" as used herein generally refers to a device, module, machine, or system capable of detecting or measuring a property or changes in its environment and sending the information or data (e.g. optical or image or acoustic data) to other electronics, frequently a computer processor. Non limiting examples of sensor types within the scope of the present invention include, but are not limited to acoustic, sound and/or vibration sensors, electric current, electric potential, magnetic and/or radio sensors, flow and/or fluid velocity sensors, optical, light, imaging and/or photon sensors, pressure sensors, thermal, heat and/or temperature sensors and position/location sensors.

The term "optical sensor" or "optical element" as used herein is meant to include photoconductive devices which convert a change of incident light into a change of resistance, photodiodes which convert an amount of incident light into an output current, phototransistors are a type of bipolar transistor where the base-collector junction is exposed to light. Optical sensors within the scope of the present invention may include: optical detector, a camera, a photodiode, a photomultiplier, an image acquisition sensor, an optical acquisition sensor, an electro-optical sensor, light detector, a photon sensor, a reflectometer, a photodetector, a spectral image sensor, and any combination thereof. This term further encompasses an optical element such as a lens, a mirror, a polarizer, an excitation filter, an emission filter, a dichroic mirror, an optical coating such as antireflective coating, an optical grating, at least one stereoscopic microscope, at least one fluorescence microscope and any combination thereof.

The term "imaging sensor" or "image sensor" or "image acquisition sensor" as used herein refers to a sensor that detects and conveys information used to make an image. Without wishing to be bound by theory, an imaging sensor conveys the variable attenuation of light waves, passed through or reflect off objects, into signals, that convey the information. The waves can be light or other electromagnetic radiation. Image sensors are used in electronic imaging devices of both analog and digital types, which include digital cameras, camera modules, camera phones, and others.

Exemplary imaging sensors within the scope of the present invention include: RGB (red, green, blue) frequency spectrum, multispectral, hyperspectral, visible light frequency range, near infrared (NIR) frequency range, infrared (IR) frequency range, monochrome, specific light wavelengths (e.g. LED or laser and/or laser and/or halogen and/or xenon and/or fluorescent), UV frequency range, a reflectometer and combinations of the aforementioned.

As used herein, the term "optical data" generally refers to data retrieved from optical media which can be stored on an optically readable medium. Examples of optical storage devices, include CD, DVD and Blu-ray discs etc. In other aspects it refers to an optically formed reproduction of an object, such as one formed by a lens or mirror. The term optical data within the context of the present invention encompass image data.

The term "image data" herein means a photographic or trace objects that represent the underlying pixel data of an area of an image element, which is created, collected and stored using image constructor devices. Image data attributes include for example, image resolution, data-point size and spectral bands. In the context of the present invention the term "computer" stands for but no limited to a machine or device that performs processes, calculations and operations based on instructions provided by a software or hardware program. The term computer also means in the context of the present invention a control unit or controller. It is designed to process and execute applications and provides a variety of solutions by combining integrated hardware and software components. The computer of the invention is configured to extract a predetermined set of feature vectors from the image data of pre-adult insects; to compute sex-characteristics of the insects based on the set of feature vectors, attributes or parameters; to generate sex-classification output and to transmit the output to the controller unit.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, and any suitable combination of the foregoing.

A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including
instructions which implement aspects of the function/act specified in the flowchart and/or block diagram block or blocks.

According to certain aspects, the method of the present invention comprises steps of applying a machine learning process with the computer implemented trained algorithm to determine the sex of pre-adult insects. Thus it is within the scope of the present invention that the algorithm (or computer readable program) is implemented with a machine learning process using a neural network with the processed data.

The term training in the context of machine learning implemented within the system of the present invention refers to the process of creating a machine learning algorithm. Training involves the use of a deep-learning framework and training dataset. A source of training data can be used to train machine learning models for a variety of use cases, from failure detection to consumer intelligence.

The neural network may compute a classification category, and/or the embedding, and/or perform clustering, for identifying sex of an individual insect at a pre-adult stage, i.e. male or female.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks herein disclosed.

As used herein, the term "classifying" may sometimes be interchanged with the term clustering or tagging, for example, when multiple insect images are analyzed, each image may be classified according to its predefined feature vectors and used to creating clusters, and/or the insect images may be embedded and the embeddings may be clustered. The term classification category may sometimes be interchanged with the term embedding, for example, the output of the trained neural network in response to an image of an insect may be one or more classification categories, or a vector storing a computed embedding. It is noted that the classification category and the embedding may be outputted by the same trained neural network, for example, the classification category is outputted by the last layer of the neural network, and the embedding is outputted by a hidden embedding layer of the neural network.

The architecture of the neural network(s) may be implemented, for example, as convolutional, pooling, nonlinearity, locally-connected, fully-connected layers, and/or combinations of the aforementioned.

It is noted that the tagging and classifying of the insects in the images or the sex-sorting characteristic targets may be manually or semi manually entered by a user (e.g., via the GUI, for example, selected from a list of available phenotypic characteristic targets), obtained as predefined values stored in a data storage device, and/or automatically computed.

The term "feature vector" refers hereinafter in the context of machine learning to an individual measurable property or characteristic or parameter or attribute of a phenomenon being observed e.g., detected by a sensor. It is herein apparent that choosing an informative, discriminating and independent feature is a crucial step for effective algorithms in pattern recognition, machine learning, classification and regression. Algorithms using classification from a feature vector include nearest neighbor classification, neural networks, and statistical techniques.

In computer vision and image processing, a feature is an information which is relevant for solving the computational task related to a certain application. Features may be specific structures in the image such as points, edges or objects. Features may also be the result of a general neighborhood operation or feature detection applied to the image. When features are defined in terms of local neighborhood operations applied to an image, a procedure commonly referred to as feature extraction is executed.

Although embodiments of the disclosure are not limited in this regard, discussions utilizing terms such as, for example, "processing", "computing", "communicating", "training", "capturing", "executing", "calculating", "feeding", "determining", "establishing", "analyzing", "checking", "tagging", "classifying", "transmitting", "exerting" or the like, may refer to operation(s) and/or process(es) of a computer, a computing platform, a computing system, a computer implemented algorithm or other electronic computing device, that manipulates and/or transforms data represented as physical (e.g., electronic) quantities within the computer's registers and/or memories into other data similarly represented as physical quantities within the computer's registers and/or memories or other information non-transitory storage medium (e.g., a memory) that may store instructions to perform operations and/or processes .

According to one embodiment, the present invention provides a device, system and method for sex-sorting of larvae and/or pupae. In some of its aspects, the system comprises a fluidic channel comprising an inlet and a number of outlets; a classification region and a separation or destruction region. The channel is configured to receive larvae and/or pupae suspended in a liquid. The larvae and/or pupae pass through a classification region where they are imaged and classified according to their sex by a processing unit. According to this classification, in some embodiments, the larvae and/or pupae are separated into different outlets, where they can then be collected. In some embodiments, the larvae and/or pupae are classified according to their sex with one class passing undamaged to the single outlet for collection, and the rest being destroyed.

It is within the scope of the present invention that a liquid, typically water, containing larvae and/or pupae enters the system, through the inlet and flows, under pressure or gravity, towards the outlet(s). The inner dimensions of the flow channel may be designed such that only a single larva or pupa passes at a time. Furthermore, these dimensions may be set so as to limit motion of the larvae and/or pupae (e.g., a narrow enough channel constricts larvae to extend parallel to the channel walls). The temperature of the liquid may be changed or controlled, namely heated (elevated) or cooled (reduced) according to the need. One such use may be to control or affect movements of the pre-adult insects during sorting or separating process (i.e. cooled to reduce wriggling movements and heated to increase wriggling movements). In one embodiment, the temperature of the liquid may be cooled so as to reduce wriggling movements. As the larvae and/or pupae pass through the classification region, they are imaged. The pictures/images are analyzed by a processing unit and the insects are classified according to their sex (male, female and undetermined).

In some embodiments, a separation region is used where each individual larva or pupa is guided to the outlet associated with its class. This may be done by leaving only the appropriate outlet open and closing the rest. Alternatively, all outlets may be left open and one or more of the following techniques may be used to guide each individual to the appropriate one: (1) acoustic radiation forces generated by an external transducer; (2) electroosmotic forces generated by electrodes (forces applied on the liquid); or (3) dielectrophoretic forces generated by electrodes (forces applied on the larva or pupa). The outlets themselves may also be designed to have different hydrodynamic resistance. Thus, when no external force is applied, the larvae and/or pupae flow to the outlet with lowest resistance.

In some embodiments, the number of individuals dispensed into each outlet channel(s) is monitored. This is particularly important when one channel is connected at its end to a container to be used for release.

In some embodiments, a destruction region, through which all individuals pass, is used. Larvae and/or pupae identified as belonging to the class that is needed for the application (e.g., males or females, according to the purpose/application for sorting the pre-adult insects), pass unharmed. The rest (also herein referred to as 'other' or 'unspecified' or 'unclassified') are destroyed. This may be done by at least one of the following means: a laser, a high -power electric field, ultrasonic blasts or by mechanically grinding or squashing the insects. In some embodiments, the number of individuals dispensed unharmed is monitored.

According to further embodiments of the present invention, an electro-optical setup next to the classification region is used to recognize the sex of the larvae and/or pupae. It includes at least one of: one or more light sources to illuminate the classification region (possibly from different directions and in different colors); one or more image sensors, to photograph the classification region (possibly from different directions); an optical setup, comprising one or more optical elements, e.g., lenses, mirrors, polarizers, excitation filters, emission filters, dichroic mirrors, optical coatings (e.g., antireflective coatings) and optical gratings, located between the light source(s) and the classification region and/or the classification region and the image sensor(s); and a connection to a processing unit. In some embodiments, the optical setup is comprised of one or more stereoscopic microscopes and/or fluorescence microscopes and the image sensors are cameras attached to the microscopes.

According to some embodiments, pictures taken by the image sensors are analyzed in the processing unit to determine the class of an individual larva or pupa. Visual attributes are used for the classification such as overall size and morphology; size and morphology of different segments; gonad size and/or morphology and/or autofluorescence; size and/or morphology and/or autofluorescence of testes and male accessory glands; and size and/or morphology and/or autofluorescence of the developing male and female secondary reproductive structures (e.g., within the anal segment).

It is further within the scope of the present invention that differences between male and female larvae/pupae may be sorted by one or more attributes including, but are not limited to, size, shape, segment size ratio, IR (Infrared) absorption, color, fluorescence, gonad disc morphology and secondary sex organ morphology.

According to further aspects of the present invention, classification may be carried out automatically using one or more of the following techniques: image processing, machine learning and trained neural network. The neural network may be a classical neural network or a deep network (deep learning network), including a convolutional neural network, or any other kind of neural network.

In other aspects of the present invention, the flow velocity of each larva and/or pupa may be monitored in order to determine when they reach the separation region and need to be guided to the appropriate outlet or reach the destruction region and need to pass freely or be destroyed. In some embodiments, this is done by taking successive images in the classification region. In some embodiments, a flow-meter or an additional camera with a wide field of view, are used for tracking the insects along the system.

Reference is now made to figures in which exemplary non-limiting embodiments are described.

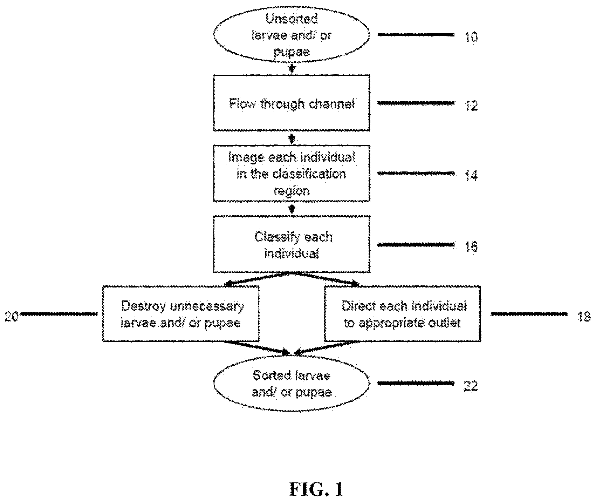

Fig. 1 illustrates a flow chart showing a generalized embodiment of the present invention. A method for male-female sorting in a flow system may begin with the provision of unsorted larvae and/or pupae as shown in step 10. Within the system, the individual larva and/or pupa flow preferably in a single-file towards the classification region, as shown in step 12. There, each individual is imaged for classification purposes as shown in step 14 of the flow chart. In step 16 each individual is classified. Classes used may include males and the rest (other, unspecified, unclassified or anything else), or males, females and the rest (other, unspecified, unclassified or anything else) or females and the rest (other, unspecified, unclassified or anything else). Next, the individual larvae and/or pupae reach a separation region, where they are guided, according to their classification, to a predefined flow outlet 18. Alternatively, if only a single class is needed, the unnecessary individuals may be destroyed in the channel 20. The outcome of the above described process is sex-sorted larvae and/or pupae 22.

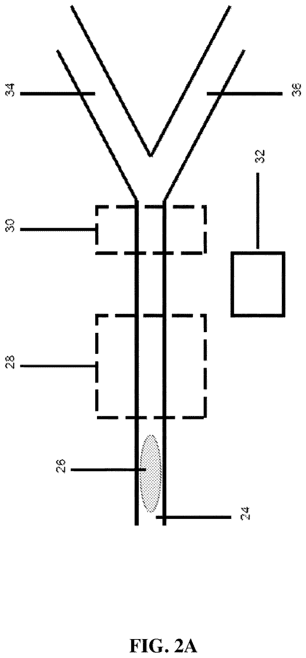

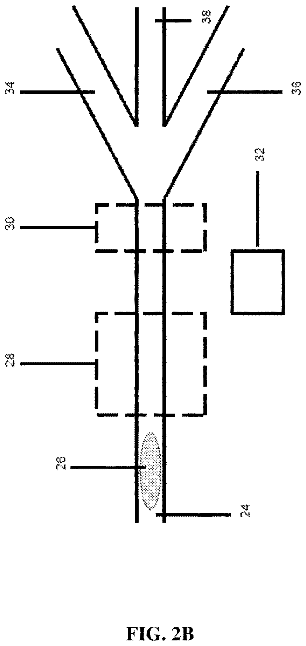

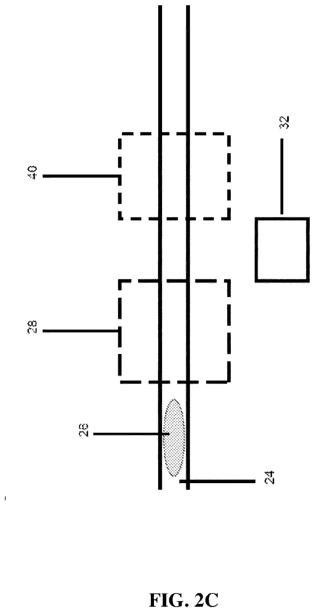

Figs 2A-C shows schematic illustrations of the larvae and/or pupae sorting device or system of the present invention. These figures show a main fluidic channel 24 through which individual larvae and/or pupae 26 flow from left to right. When the larvae and/or pupae pass through the classification region 28 they are illuminated, and imaged using an optical system or sensor. They then continue through the main channel until reaching a separation region 30. In the separation region 30 the individual larvae and/or pupae are guided to different channels according to their classification performed by a processing unit 32 communicably connected to the system or device. Classification attributes may include, but are not limited to size, shape, segment size ratio, IR (Infrared) absorption, color, fluorescence, gonad disc morphology and secondary sex organ morphology. Fig. 2A shows a schematic illustration, where the individual larvae and/or pupae are guided to either a 'male-only' or 'female-only' channel 34 or an 'anything else' channel 36. Fig. 2B shows a schematic illustration, where the individual larvae and/or pupae may be guided to either a 'male-only' channel 34, a 'female-only' channel 38 or to an 'anything else' channel 36. Fig. 2C shows a schematic illustration, where after the classification region only a single class of larvae and/or pupae continue flowing, undamaged, through the main channel. The rest are destroyed in a destruction region 40.

According to one embodiment the present invention provides a system for sex separation of pre-adult insects comprising: (a) a fluidic channel having an inner space and an outer wall, the fluidic channel comprising an inlet and at least one outlet, wherein the channel is configured to allow flow of pre-adult insects suspended in liquid media; (b) a processor; and, (c) a controller in-communication with the processor; wherein the fluidic channel comprises a classification region, and the classification region comprises an electro-optical module in-communication with the processor, the electro-optical module comprises at least one sensor configured to acquire optical data of an individual pre-adult insect and to transmit the optical data to the processor, further wherein the processor is configured to process the acquired optical data, to classify the pre-adult insect based on the acquired optical data, and to instruct the controller to sort the pre-adult insect based on the classification of the pre-adult insect.

According to a further embodiment of the system of the present invention, the inner space of the fluidic channel is adjustable to the dimensions of a single pre-adult insect.

According to a further embodiment of the system of the present invention, the fluidic channel is designed to allow passage of single pre-adult insect at a time.

According to a further embodiment the system is further configured to control movement of the pre-adult insect when passing through the fluidic channel.

According to a further embodiment of the system of the present invention, the flow of the pre-adult insect suspended in the liquid media through the fluidic channel is a passive flow.

According to a further embodiment of the system of the present invention, the flow of the pre-adult insect suspended in the liquid media through the fluidic channel is an active flow.

According to a further embodiment of the system of the present invention, the flow is driven by a pump, vacuum, pressurizing means such as pressurized gas, gravity forces or any combination thereof.

According to a further embodiment the system comprises more than one outlet, wherein each outlet is independently operated by the controller.

According to a further embodiment of the system of the present invention, each of the one or more outlets is associated with a classification category selected from the group consisting of male insect, female insect and other.

According to a further embodiment of the system of the present invention, the at least one outlet having an "open" and a "closed" configuration.

According to a further embodiment of the system of the present invention, the outlet is operably coupled to a container.

According to a further embodiment of the system of the present invention, the fluidic channel further comprises a separation region, wherein the separation region is located between the classification region and the at least one outlet; and wherein the separation region comprises at least one insect-guiding tool configured to guide the pre-adult insect to an outlet associated with the insect classification.

According to a further embodiment of the system of the present invention, the insect-guiding tool is selected from the group consisting of one or more valves, a transducer configured to generate acoustic radiation forces, electrodes configured to apply electroosmotic forces to the liquid media, electrodes configured to apply dielectrophoretic forces to the pre-adult insect and any combination thereof.

According to a further embodiment of the system of the present invention, the fluidic channel further comprises a destruction region; wherein the destruction region comprises at least one insect-destroying tool in-communication with the controller; and wherein the insect-destroying region has an "on" and "off' configurations operated by the controller.

According to a further embodiment of the system of the present invention, the at least one insect-destroying tool is selected from the group consisting of a laser beam, a high-power electric field, ultrasonic blasts, a grinding tool, a squashing tool and any combination thereof.

According to a further embodiment of the system of the present invention, the controller is configured to execute functions selected from the group consisting of: receiving instructions from the processor, controlling flow rate, switching on the insect-guiding tool, switching off the insect-guiding tool, switching on the insect-destroying tool, switching off the insect-destroying tool, switching configuration of the outlet from "open" to "close", and, switching configuration of the outlet from "closed" to "open".

According to a further embodiment of the system of the present invention, the sensor is selected from the group consisting of an optical detector, a camera, a photodiode, a photomultiplier, an image acquisition sensor, an optical acquisition sensor, an electro-optical sensor, light detector, a photon sensor, a reflectometer, a photodetector, a spectral image sensor, and any combination thereof.

According to a further embodiment of the system of the present invention, the sensor is an imaging sensor selected from the group of consisting of RGB frequency spectrum, multispectral, hyperspectral, visible light frequency range, near infrared (NIR) frequency range, infrared (IR) frequency range, monochrome, specific light wavelengths (e.g. LED and/or laser and/or halogen and/or xenon and/or fluorescent), UV frequency range and any combination thereof.

According to a further embodiment of the system of the present invention, the electro-optical module of the classification region comprises at least one of: one or more light sources configured to illuminate the classification region, one or more image sensors, one or more acoustic sources configured to generate sound waves that pass through the classification region, at least one optical element and an internal control unit in-communication with the processor.

According to a further embodiment of the system of the present invention, the electro-optical module comprises more than one light source, wherein each light source is configured to emit light with predetermined spectrum and/or intensities.

According to a further embodiment of the system of the present invention, the electro-optical module is configured to obtain multiple images of the pre adult insect from different angles.

According to a further embodiment of the system of the present invention, the image acquisition sensor is a high-frame rate.

According to a further embodiment of the system of the present invention, the light source illumination is stroboscopic or pulsed.

According to a further embodiment of the system of the present invention, the optical element is selected from the group consisting of a lens, a mirror, a polarizer, an excitation filter, an emission filter, a dichroic mirror, an optical coating such as antireflective coating, an optical grating and any combination thereof.

According to a further embodiment of the system of the present invention, the optical element comprises at least one stereoscopic microscope, at least one fluorescence microscope, or a combination thereof.

According to a further embodiment of the system of the present invention, the sensor is attached to the at least one microscope.

According to a further embodiment, the system of the present invention further comprises a sensor in-communication with the controller, wherein the controller is further configured to control the amount of insect individuals at the entrance to the fluidic channel based on the data acquired by the sensor, to thereby allow passage of single pre-adult insect in a time to the fluidic channel.

According to a further embodiment of the system of the present invention, the processor is configured to perform functions selected from receiving the optical data of an individual pre-adult insect acquired by the sensor of the electro-optical module, processing the optical data, extracting a set of parameters indicative of the sex of the pre-adult insect from the optical data, classifying the pre-adult insect based on the set of parameters extracted from the optical data, providing instructions to the controller based on the classification of the pre-adult insect.

According to a further embodiment of the system of the present invention, the processor is configured to classify the individual pre-adult insect into at least two classes selected from the group consisting of male and non-male.

According to a further embodiment of the system of the present invention, the processor is configured to classify the individual pre-adult insect into at least two classes selected from the group consisting of female and non-female.

According to a further embodiment of the system of the present invention, the processor is configured to classify the individual pre-adult insect into three classes selected from the group consisting of male, female, and other.

According to a further embodiment of the system of the present invention, the parameters indicative of the sex of the pre-adult insect are selected from the group consisting of overall size of the pre-adult insect, morphology of the pre-adult insect, shape, segment size ratio, IR (Infrared) absorption, color, fluorescence, gonad disc morphology, secondary sex organ morphology, gonad size, gonad morphology, gonad autofluorescence, size of testes, size of male accessory glands, morphology of testes, morphology of male accessory glands, autofluorescence of testes, autofluorescence of male accessory glands, size of the developing male and female primary or secondary reproductive structures, primitive sex organs, morphology of the developing male and female primary or secondary reproductive structures, autofluorescence of the developing male and female primary or secondary reproductive structures and any combination thereof.

According to a further embodiment of the system of the present invention, the classification region further comprises a glass capillary through which the pre-adult insect flows.

According to a further embodiment, the system of the present invention further comprises a thermostat optionally operated by the controller, wherein the thermostat is configured to control the temperature in the fluidic channel.

According to a further embodiment of the system of the present invention, the liquid media is water or isotonic aqueous solution.

According to a further embodiment of the system of the present invention, the sorting rate is about 0.1 to 100 pre-adult insects per second.

According to a further embodiment of the system of the present invention, the inner space of the fluidic channel has the size in the range of 0.1 mm to 10 mm.

According to a further embodiment of the system of the present invention, the sorting accuracy is in the range of 50% to 100%, particularly 80% to 100%, more particularly at least 90%.

According to a further embodiment of the system of the present invention, the sorting specificity is in the range of 50% to 100%, particularly 80% to 100%, more particularly at least 90%.

According to a further embodiment of the system of the present invention, the sorting selectivity is in the range of 50% to 100%, particularly 80% to 100%, more particularly at least 90%.

According to a further embodiment of the system of the present invention, the pre-adult insect is a larva or a pupa or a nymph.

According to a further embodiment of the system of the present invention, the pre-adult insect type is selected from the group of Endopterygota consisting of: Coleoptera (Beetles), Diptera (Flies), Hymenoptera (Ants, bees, sawflies and wasps), Lepidoptera (Butterflies, and moths), Mecoptera (Scorpionflies),
Megaloptera (Alderflies, dobsonflies, and fishflies), Miomoptera, Neuroptera (Lacewings, antiions), Raphidioptera (Snakeflies), Siphonaptera (Fleas), Strepsiptera (Twisted-winged parasites) and Trichoptera (Caddisflies).

According to a further embodiment of the system of the present invention, the pre-adult insect species is selected from the group consisting of: (a) Diptera species, such as from the family Culicidae, e.g. Culex pipiens, Aedes Aegypti, Aedes albopictus, Anopheles gambiae; from the family Drosophilidae, e.g. Drosophila melanogaster, Drosophila suzukii; from the family Tephritidae, e.g. Bactrocera tryoni, Anastrepha fraterculus, Anastrepha ludens; from the family Stratiomyidae, e.g. Hermetia illucens; from the family Syrphidae, e.g. Eristalis tenax; (b) Lepidoptera species, such as from the family Plutellidae, e.g. Plutella xylostella; from the family Pyralidae, e.g. Plodia interpunctella; and (c) Coleoptera species, such as from the family Tenebrionidae, e.g. Tribolium castaneum.

According to a further embodiment, the system of the present invention further comprises a container in fluid connection with the fluidic channel, the container contains unsorted pre-adult insects suspended in a liquid media, the container has an outlet configured to allow passage of a single pre-adult insect at a time to the fluidic channel, the container further comprises pressurizing means, such as pressurized gas, vacuum, gravity forces and/or one or more pumps to drive a flow of the unsorted pre adult insects towards the outlet of the container to the fluidic channel.

It is further within the scope of the present invention to provide a container in fluid connection with fluidic channel for sex separation of pre-adult insects, the container contains unsorted pre-adult insects suspended in a liquid media, the container has an outlet configured to allow passage of a single pre adult insect at a time to the fluidic channel, the container further comprises pressurizing means, such as pressurized gas, vacuum, gravity forces and/or one or more pumps to drive a flow of unsorted pre-adult insects towards the outlet of the container to the fluidic channel.

It is further within the scope of the present invention to provide a computer implemented method of sex separation of pre-adult insects comprising: (a) providing the system for sex separation of pre-adult insects as defined in any of the above; (b) streaming the pre-adult insects suspended in the liquid media through the inlet into the fluidic channel towards the classification region; (c) acquiring optical data of the individual pre-adult insect at the classification region by one or more sensors of the electro-optical module; (d) transmitting the optical data of the individual pre-adult insect to the processor; (e) processing the optical data and classifying the individual pre-adult insect based on a set of parameters indicative of the sex of the pre-adult insect extracted from the optical data; (f) providing instructions to the controller based on the classification of the individual pre-adult insect; and, (g) sorting the pre-adult insect according to the instructions received by the controller.

It is further within the scope of the present invention to provide the method as defined above, wherein the step of sorting of the pre-adult insect according to the instructions received by the controller comprises at least one of: controlling flow rate, controlling sorting rate, switching on the insect-guiding tool, switching off the insect guiding tool, switching on the insect-destroying tool, switching off the insect-destroying tool, switching the configuration of the outlet from "open" to "closed", and, switching the configuration of the outlet from "closed" to "open".

It is further within the scope of the present invention to provide the method as defined in any of the above, wherein the pre-adult insects are classified into two classes, wherein the two classes are male and non-male.

It is further within the scope of the present invention to provide the method as defined in any of the above, wherein the pre-adult insects are classified into two classes, wherein the two classes are female and non-female.

It is further within the scope of the present invention to provide the method as defined in any of the above, wherein the pre-adult insects are classified into three classes, wherein the three classes are male, female and other.

It is further within the scope of the present invention to provide the method as defined in any of the above, wherein the pre-adult individuals classified as non-male are directed to the destruction region to be destroyed by the at least one destroying tool.

It is further within the scope of the present invention to provide the method as defined in any of the above, wherein the pre-adult individuals classified as non-female are directed to the destruction region to be destroyed by the at least one destroying tool.

It is further within the scope of the present invention to provide the method as defined in any of the above, wherein the pre-adult insect is a larva or a pupa or a nymph.

It is further within the scope of the present invention to provide the method as defined in any of the above, wherein the pre-adult insect type is selected from the group of Endopterygota consisting of: Coleoptera (Beetles), Diptera (Flies), Hymenoptera (Ants, bees, sawflies and wasps), Lepidoptera (Butterflies, and moths), Mecoptera (Scorpionflies), Megaloptera (Alderflies, dobsonflies, and fishflies), Miomoptera, Neuroptera (Lacewings, antiions), Raphidioptera (Snakeflies), Siphonaptera (Fleas), Strepsiptera (Twisted-winged parasites) and Trichoptera (Caddisflies).

It is further within the scope of the present invention to provide the method as defined in any of the above, wherein the pre-adult insect species is selected from the group consisting of: (a) Diptera species, such as from the family Culicidae, e.g. Culex pipiens, Aedes Aegypti, Aedes albopictus, Anopheles gambiae; from the family Drosophilidae, e.g. Drosophila melanogaster, Drosophila suzukii; from the family Tephritidae, e.g. Bactrocera tryoni, Anastrepha fraterculus, Anastrepha ludens; from the family Stratiomyidae, e.g. Hermetia illucens; from the family Syrphidae, e.g. Eristalis tenax; (b) Fepidoptera species, such as from the family Plutellidae, e.g. Plutella xylostella; from the family Pyralidae, e.g. Plodia interpunctella; and (c) Coleoptera species, such as from the family Tenebrionidae, e.g. Tribolium castaneum.

It is further within the scope of the present invention to provide the method as defined in any of the above, further comprises steps of adjusting the dimensions of the inner space of the fluidic channel to allow passage of a single pre adult insect at a time.

It is further within the scope of the present invention to provide the method as defined in any of the above, further comprises steps of adjusting the dimensions of the inner space of the fluidic channel such that a single pre-adult insect extends parallel to the channel walls.

It is further within the scope of the present invention to provide the method as defined in any of the above, further comprises steps of pressurizing or directing by gravity forces unsorted pre-adult insects towards the outlet of the fluidic channel.

It is further within the scope of the present invention to provide the method as defined in any of the above, further comprises steps of reducing or elevating the temperature of the liquid media so as to control wriggling movements of the pre adult insects flowing within the fluidic channel.

It is further within the scope of the present invention to provide the method as defined in any of the above, further comprises steps of reducing the temperature of the liquid media so as to reduce wriggling movements of the pre-adult insects flowing within the fluidic channel.

It is further within the scope of the present invention to provide the method as defined in any of the above, comprises steps of acquiring optical data by a sensor selected from the group consisting of an optical detector, a camera, a photodiode, a photomultiplier, an image acquisition sensor, an electro-optical sensor, an optical acquisition sensor, a light detector, a photon sensor, a reflectometer, a photodetector, a spectral image sensor, and any combination thereof.

It is further within the scope of the present invention to provide the method as defined in any of the above, wherein the electro-optical module of the classification region comprises at least one of: one or more light sources configured to illuminate the classification region, one or more acoustic sources configured to generate sound waves that pass through the classification region, one or more image sensors, at least one optical element and an internal control unit in-communication with the processor.

It is further within the scope of the present invention to provide the method as defined in any of the above, wherein the classifying comprises using a trained neural network.

It is further within the scope of the present invention to provide the method as defined in any of the above, wherein the step of processing comprises steps of computing the optical data using computer implemented algorithm trained to generate output based on the optical data.

It is further within the scope of the present invention to provide the method as defined in any of the above, wherein the computer implemented algorithm is trained to generate output based on predetermined feature vectors or attributes extracted from the optical data.

It is further within the scope of the present invention to provide the method as defined in any of the above, wherein the method comprises steps of implementing with the algorithm a training process according to a training dataset comprising a plurality of training images of a plurality of pre-adult insects captured by the at least one imaging sensor, wherein each respective training image of the plurality of training images is associated with the sex determination of the pre-adult insect depicted in the respective training image.

It is further within the scope of the present invention to provide the method as defined in any of the above, wherein the training process comprises steps of (a) capturing images of pre-adult insects using an imaging sensor; (b) classifying images into classification categories by applying a tag associated with parameters or attributes indicative of the sex of the pre-adult insect extracted from the optical data; and (c) applying a computer vision algorithm to determine a set of feature vectors associated with each classification category.

It is further within the scope of the present invention to provide the method as defined in any of the above, further comprises steps of applying a machine learning process with the computer implemented trained algorithm to determine the sex of the imaged pre-adult insect.

It is further within the scope of the present invention to provide the method as defined in any of the above, wherein the algorithm is implemented with a machine learning process using a neural network with the processed data.

It is further within the scope of the present invention to provide the method as defined in any of the above, wherein the machine learning process comprises computing by the at least one neural network, a tag of at least one classification category for the at least one pre-adult insect, wherein the tag of at least one classification category is computed at least according to weights of the at least one neural network, wherein the at least one neural network is trained according to a training dataset comprising a plurality of training images of a plurality of pre-adult insects captured by the at least one imaging sensor, wherein each respective training image of the plurality of training images is associated with the tag of at least one classification category of at least one pre-adult insect depicted in the respective training image; and generating according to the tag of at least one classification category, instructions for execution by the controller.

It is further within the scope of the present invention to provide the method as defined in any of the above, wherein the sensor is configured for image capturing and processing, with or without using Artificial Intelligence (Al) and/or machine learning and/or neural networks.

It is further within the scope of the present invention to provide a computer implemented algorithm comprising code to perform the steps of the method as defined in any of the above.

According to a further embodiment, the computer implemented algorithm as defined above, wherein the algorithm is a machine learning algorithm.

According to a further embodiment, the computer implemented algorithm as defined in any of the above, wherein the machine learning algorithm uses verified training data.

In order to understand the invention and to see how it may be implemented in practice, a plurality of preferred embodiments will now be described, by way of non-limiting example only, with reference to the following examples.

### EXAMPLE 1

### A system for sex-sorting insects

An exemplified sex-sorting system essentially is based on the following: Mosquito larvae are kept in their natural environment, namely, water. Unsorted larvae flow, under pressure or gravity, towards the outlets of the system. The inner dimensions of the flow channel were chosen such that only a single larva can pass at a time. Furthermore, these dimensions constrict the larvae to extend parallel to the channel walls. The temperature of the liquid may also be cooled so as to reduce wriggling movements. As the larvae pass through a classification region, they are imaged.

### Additional optional features:

Thickness of tubes and capillary may vary according to the larvae/ pupae size of the insect being sorted and the developmental stage at the time the sorting is being performed. For example, for Culex pipiens and Aedes albopictus larvae, the inner dimension size ranges from 0.3 to 3mm.

Aqueous medium used to flow the larvae/pupae (typically water) may be cooled in order to reduce their movements during the sorting process.

The unsorted larvae/pupae may be kept dispersed in the liquid before entering the sorting system through agitation. This may help having the larvae/pupae enter the system one by one and at a constant rate.

A sensor at the entrance to the system may be used to detect if two or more larvae/pupae have entered it at close proximity. If this occurs, the larvae are diverted outside to prevent possible mistakes (such as clogging or identifying multiple larvae/pupae as one).

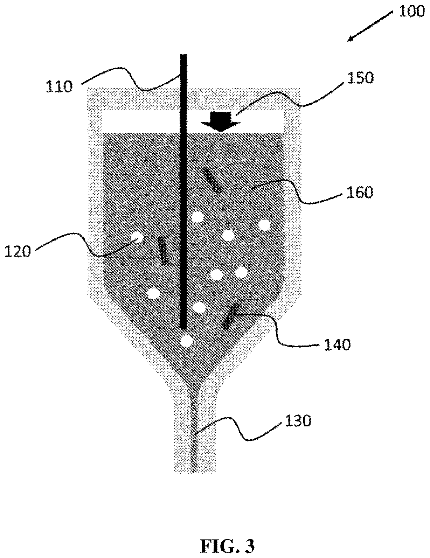

Pressurized gas may be used to mix the larvae/pupae and drive them through the system (see Fig. 3).

Imaging with a color camera enables to improve differentiation by using the different absorption characteristics of the different organs.

Imaging from 2 or more distinct angles in order to improve detection of objects of interest (e.g., gonads should be visible from one of the angles). This may be done using 2 or more cameras or by using a single camera and directing the light from different regions on different parts of the imaging sensor.

High frame rate (>30 frames per second) and stroboscopic illumination may be used in order to image the flowing larvae without having to stop them before capturing the image.

In the imaging region/unit, the larvae/ pupae may flow through a glass capillary to improve image quality. A square or rectangular capillary may be used, for a more uniform background and to remove spherical aberrations.

A flow rate of ~ 20 ml per minute and larvae density of ~ 1.5 per ml is aimed for example for mosquito larvae sex-separation.

Both male and female primary and secondary primitive (not fully developed) sex organs can be used.

### EXAMPLE 2

### Sex- sorting of insects by the system of the present invention

At the first step, the survival and eclosion (or adult emergence from pupae) of mosquitoes that were sorted by the system as described in any of Example 1 and Figs 1 and 2, was tested; no adverse effects to the mosquitoes have been found.

In addition, it was demonstrated that the sex of larvae of two species belonging to two distinct families, namely Culex pipiens and Aedes albopictus can be reliably recognized. This was performed by observing the larvae under a light microscope, determining the sex, and growing each larva separately to adulthood, a stage when sex is clearly distinguishable (almost unmistakable).

Images of male and female Culex pipiens and Aedes albopictus larvae show that the primitive sex organs are readily discernible, even under low magnification (x0.5-xl0) of a light microscope, at the L3 stage of the larval developmental cycle.

It was tested that the sex of each larva can be predicted, primarily based on the presence or absence of the male gonads. According to further aspects of the present invention, these sex organs (including primitive sex organs) may be detected even at earlier developmental stages.

To test the above predictions, each individual larva was grown till adulthood, where the sex is easily discernible. Table 1 presents results obtained after analyzing over 120 larvae.

**Table 1: Sex-sorting results**

| | Specificity (%) | Sensitivity (%) |
|---|---|---|
| *Culex pipiens* | 100 | -90 |
| *Aedes albopictus* | 100 | -85 |

It was found that the specificity was 100% (i.e. no females recognized as males). The sensitivity for the Culex pipiens was higher than 90% and for Aedes albopictus about 85% (i.e. 10 to 15% of the males were missed, respectively).

It was further demonstrated that by using the system for sex separation of Drosophila melanogaster the same process is even more reliable, showing almost 100% sensitivity with 100% specificity.

The above results show that the process for sex-sorting as herein disclosed is suitable or applicable to most insects with a distinct larval stage. These include, for example mosquitoes, flies, moths and beetles. In order to adjust the system, e.g. as described in Example 1 and Figs 1 and 2, to a predetermined insect type or species, the following steps should be performed:
1. Adjusting the geometry of the flow system to fit the size of the predetermined larvae species.
2. Obtaining images of said larvae using the system of the present invention to ensure that the sexes can be reliably differentiated.
3. Training a machine learning algorithm to differentiate between the sexes of the predetermined larvae/pupae species.

For insects whose larvae do not naturally live in water, the larvae should be transferred from growth media to water (or any other isotonic aqueous solution). This can be performed by physical sieving of the larvae from the growth media, floating the larvae out using viscous solution or by utilizing the natural/biological behavior of the insect (for example, some fly larvae drop out of the food close to pupation so if the food is held above the water they will transfer on their own, dropping out prior to pupation).

Non limiting examples of insects included within the scope of the present invention, or in other words, insects that can be sex-sorted by the system and method of the present invention include: Plutella xylostella, Bactrocera tryoni, Drosophila suzukii, Hermetia illucens, Anastrepha fraterculus, Anastrepha ludens, Tribolium castaneum and Plodia interpunctella.

With respect to the imaging platform, image quality was compared between an inverted Feica DM IRE2 microscope (xIO, NA 0.3 objective), an Olympus SZH10 stereo microscope and a Raspberry Pi with VI camera module and macro lens. No significant differences were found between the three systems, and the gonads, when visible, were clearly discernible in all three.

A trained machine learning (ME) code is applied and performs 100% separation, as achieved manually. In a specific example, a neural network is trained with a small corpus of 40 larvae and showed at least 90% accuracy. If needed, precision can be increased by running the sorted larva/pupae of interest again through the system.

To summarize a semi-automated or automated system for sex-sorting of mosquito larvae was developed by the inventors. In some embodiments, the system performs the following process:

### Receives a container with water and unsorted larvae

The larvae pass one-by-one into a flow system

The flowing larvae are imaged

After imaging, the system can switch the larvae to the appropriate outlet (male or female container) according to a predesigned computer algorithm.

The larvae are not harmed by the process

It can be concluded by the experimental results that:

No damage was done to the larvae that were sex sorted by the system of the present invention and all of the larvae developed properly to adulthood.

Sorting rates of 0.1 to 100 pre-adult insects per second, particularly 0.5 to 50, more particularly 1 to 10, even more particularly 1-2 larvae per second are achievable.

### EXAMPLE 3

### A container with unsorted larvae and/or pupae

Reference is now made to Fig. 3 presenting an exemplified container 100 filled with unsorted larvae 140 and water 160 which, in some embodiments is connected to the sorting system of the present invention. Such a container may have a narrow outlet 130 through which the larvae 140 flow into the sorting system (e.g. as described in Example 1 and Figs 1 and 2). Pressurized gas (typically air) 120 may be released in the container, through a tube 110 immersed in the water. Gas pressure 150 (generated by any acceptable means known in the relevant art) pushes or drives the larvae 140 with the water 160 towards the outlet 130. It also mixes the larvae 140 in the container by bubbling the water. This gentle stirring disperses the larvae 140 in the container without harming them, overcoming their self-movement.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

Unless otherwise defined, all technical and scientific terms used herein have the same meanings as are commonly understood by one of ordinary skill in the art to which this invention belongs. Although methods similar or equivalent to those described herein can be used in the practice or testing of the present invention, suitable methods are described herein

It will be appreciated by persons skilled in the art that the present invention is not limited to what has been particularly shown and described hereinabove. Rather the scope of the present invention is defined by the appended claims.

## Claims

1. A system for sex separation of pre-adult insects comprising:
a) fluidic channel (24) having an inner space and an outer wall, said fluidic channel comprising an inlet and at least one outlet (34, 36), each one of said one or more outlets is associated with a classification category selected from the group consisting of male insect, female insect and other, wherein said channel is configured to allow flow of pre-adult insects (26) suspended in liquid media;
b) a processor; and,
c) a controller in-communication with said processor;
wherein said fluidic channel comprises a classification region (28), and the classification region comprises an electro-optical module in-communication with the processor, said electro-optical module comprises at least one sensor configured to acquire optical data of an individual pre-adult insect while suspended in liquid media and flowing in said fluidic channel, and to transmit said optical data to the processor, further wherein the processor is configured to process the acquired optical data, to classify said pre-adult insect based on the acquired optical data, and to instruct the controller to sort said pre-adult insect based on the classification of said pre-adult insect.

2. The system of claim 1, wherein said fluidic channel comprises more than one outlet, wherein each outlet is independently operated by the controller; the configuration of the outlet switches from 'open' to 'closed', and from 'closed' to 'open'.

3. The system of claim 1 or 2, wherein the fluidic channel further comprises a separation region (30), wherein said separation region is located between the classification region and the plurality of outlets; wherein said separation region comprises at least one insect-guiding tool configured to guide said pre-adult insect to an outlet associated with the insect classification, and wherein the insect-guiding tool is selected from the group consisting of one or more valves, a transducer configured to generate acoustic radiation forces, electrodes configured to apply electroosmotic forces to the liquid media, electrodes configured to apply dielectrophoretic forces to the pre-adult insect and any combination thereof.

4. The system of any one of claims 1 to 3, wherein the fluidic channel further comprises a destruction region; wherein said destruction region comprises at least one insect--destroying tool in-communication with the controller; and wherein said insect--destroying region has an "on" and "off' configurations operated by the controller.

5. The system of any one of claims 1 to 4, wherein the electro-optical module of the classification region comprises at least one of: one or more light sources configured to illuminate the classification region by emitting light with predetermined spectrum and/or intensities, one or more image sensors, one or more acoustic sources configured to generate sound waves that pass through the classification region, at least one optical element and an internal control unit in-communication with the processor, wherein the electro-optical module is configured to obtain multiple images of the pre-adult insect from different angles, and wherein the light source illumination is stroboscopic or pulsed.

6. The system of any one of claims 1 to 5, further comprising a sensor in communication with the controller, wherein the controller is further configured to control the amount of insect individuals at the entrance to the fluidic channel based on the data acquired by said sensor, to thereby allow passage of single pre-adult insect in a time to the fluidic channel.

7. The system of any one of claims 1 to 6, wherein the processor is configured to perform functions selected from receiving the optical data of an individual pre-adult insect acquired by the sensor of the electro-optical module, processing the optical data, extracting a set of parameters indicative of the sex of the pre-adult insect from the optical data, classifying the pre-adult insect based on the set of parameters extracted from the optical data, providing instructions to the controller based on the classification of the pre adult insect.

8. The system of claim 7, wherein the parameters indicative of the sex of the pre-adult insect are selected from the group consisting of overall size of the pre adult insect, morphology of the pre-adult insect, shape, segment size ratio, IR (Infrared) absorption, color, fluorescence, gonad disc morphology, secondary sex organ morphology, gonad size, gonad morphology, gonad autofluorescence, size of testes, size of male accessory glands, morphology of testes, morphology of male accessory glands, autofluorescence of testes, autofluorescence of male accessory glands, size of the developing male and female primary or secondary reproductive structures, primitive sex organs, morphology of the developing male and female primary or secondary reproductive structures, autofluorescence of the developing male and female primary or secondary reproductive structures and any combination thereof.

9. The system of any of claims 1 to 8, wherein said system further comprises a container in fluid connection with a fluidic channel for sex separation of pre-adult insects, said container contains unsorted pre-adult insects suspended in a liquid media, and having an outlet configured to allow passage of a single pre adult insect at a time to the fluidic channel, said container further comprises pressurizing means, such as pressurized gas, vacuum, gravity forces and/or one or more pumps to drive a flow of unsorted pre-adult insects towards the outlet of the container to the fluidic channel.

10. A computer implemented method of sex separation of pre-adult insect comprising:
a) providing the system for sex separation of pre-adult insects of any one of claims 1 to 9;
b) streaming the pre-adult insects suspended in the liquid media through the inlet into the fluidic channel towards the classification region;
c) acquiring optical data of the individual pre-adult insect at the classification region by one or more sensors of the electro-optical module;
d) transmitting the optical data of the individual pre-adult insect to the processor;
e) processing the optical data and classifying the individual pre-adult insect based on a set of parameters indicative of the sex of the pre-adult insect extracted from the optical
data;
f) providing instructions to the controller based on the classification of the individual pre-adult insect; and
g) sorting the pre-adult insect according to the instructions received by the controller.

11. The method of claim 10, wherein the pre-adult insects are classified into at least two classes selected from a group consisting of:
male and non-male, or female and non-female.

12. The method of any one of claims 10, 11 wherein the pre-adult insect is a larva or a pupa or a nymph.

13. The method of any one of claims 10 to 12, wherein the pre-adult insect type is selected from the group of Endopterygota consisting of: Coleoptera (Beetles), Diptera (Flies), Hymenoptera (Ants, bees, sawflies and wasps), Lepidoptera (Butterflies, and moths), Mecoptera (Scorpionflies), Megaloptera (Alderflies, dobsonflies, and fishflies), Miomoptera, Neuroptera (Lacewings, antiions), Raphidioptera (Snakeflies), Siphonaptera (Fleas), Strepsiptera (Twisted-winged parasites) and Trichoptera (Caddisflies), and wherein the pre-adult insect species is selected from the group consisting of: a) Diptera species, such as from the family Culicidae, e.g. Culex pipiens, Aedes Aegypti, Aedes albopictus, Anopheles gambiae; from the family Drosophilidae, e.g. Drosophila melanogaster, Drosophila suzukir, from the family Tephritidae, e.g. Bactrocera tryoni, Anastrepha fraterculus, Anastrepha ludens; from the family Stratiomyidae, e.g. Hermetia illucens from the family Syrphidae, e.g. Eristalis tenax b) Fepidoptera species, such as from the family Plutellidae, e.g. Plutella xylostella from the family Pyralidae, e.g. Plodia interpunctella c) Coleoptera species, such as from the family Tenebrionidae, e.g. Tribolium castaneum.

14. The method of any one of claims 10 to 13, further comprises steps of reducing or elevating the temperature of the liquid media so as to control wriggling movements of the pre-adult insects flowing within the fluidic channel.

15. The method of any one of claims 10 to 14, wherein said classifying comprises using a trained neural network.

## Patentansprüche

1. Ein System zur Geschlechtertrennung von präadulten Insekten, das Folgendes umfasst:
a) einen Strömungskanal (24)
mit einem Innenraum und einer Außenwand, wobei der genannte Strömungskanal einen Einlass und mindestens einen Auslass (34, 36) umfasst, wobei jeder der einen oder mehreren Auslässe einer Klassifizierungskategorie zugeordnet ist, die aus der Gruppe ausgewählt ist, die aus einem männlichen Insekt, einem weiblichen Insekt und anderen besteht, wobei der genannte Kanal so konfiguriert ist, dass er einen Fluss von präadulten Insekten (26) ermöglicht, die in einem flüssigen Medium suspendiert sind;
b) einen Prozessor; und,
c) eine mit dem genannten Prozessor kommunizierende Steuereinheit;
wobei der genannte Strömungskanal einen Klassifizierungsbereich (28) umfasst und der Klassifizierungsbereich ein mit dem Prozessor kommunizierendes elektrooptisches Modul umfasst, wobei das genannte elektro-optische Modul mindestens einen Sensor umfasst, der so konfiguriert ist, dass er optische Daten eines einzelnen präadulten Insekts erfasst, während es in einem flüssigen Medium suspendiert ist und in dem genannten Strömungskanal fließt, und die optischen Daten an den Prozessor überträgt, wobei der Prozessor ferner so konfiguriert ist, dass er die erfassten optischen Daten verarbeitet, das genannte präadulte Insekt auf der Grundlage der erfassten optischen Daten klassifiziert und die Steuereinheit anweist, das genannte präadulte Insekt auf der Grundlage der Klassifizierung des genannten präadulten Insekts zu sortieren.

2. Das System nach Anspruch 1, wobei der genannte Strömungskanal mehr als einen Auslass umfasst, wobei jeder Auslass unabhängig von der Steuereinheit betrieben wird; die Konfiguration des Auslasses schaltet von "offen" auf "geschlossen" und von "geschlossen" auf "offen" um.

3. Das System nach Anspruch 1 oder 2, wobei der Strömungskanal ferner einen Trennbereich (30) umfasst, wobei der genannte Trennbereich zwischen dem Klassifizierungsbereich und der Vielzahl von Auslässen angeordnet ist; wobei der genannte Trennbereich mindestens ein insektenführendes Werkzeug umfasst, das so konfiguriert ist, dass es das genannte präadulte Insekt zu einem Auslass führt, der mit der Insektenklassifizierung verbunden ist, und wobei das insektenführende Werkzeug aus der Gruppe ausgewählt ist, die aus einem oder mehreren Ventilen, einem Wandler, der so konfiguriert ist, dass er akustische Strahlungskräfte erzeugt, Elektroden, die so konfiguriert sind, dass sie elektroosmotische Kräfte auf das flüssige Medium ausüben, Elektroden, die so konfiguriert sind, dass sie dielektrophoretische Kräfte auf das präadulte Insekt ausüben, und jeder Kombination davon besteht.

4. Das System nach einem der Ansprüche 1 bis 3, wobei der Strömungskanal ferner einen Zerstörungsbereich umfasst; wobei der Zerstörungsbereich mindestens ein mit der Steuereinheit kommunizierendes insektenzerstörendes Werkzeug umfasst; und wobei der insektenzerstörende Bereich eine von der Steuereinheit betriebene "Ein"- und "Aus"-Konfiguration aufweist.

5. Das System nach einem der Ansprüche 1 bis 4, wobei das elektro-optische Modul des Klassifizierungsbereichs mindestens eines der folgenden Elemente umfasst: eine oder mehrere Lichtquellen, die so konfiguriert sind, dass sie den Klassifizierungsbereich durch Emittieren von Licht mit vorbestimmtem Spektrum und/oder Intensitäten beleuchten, ein oder mehrere Bildsensoren, ein oder mehrere akustische Quellen, die so konfiguriert sind, dass sie Schallwellen erzeugen, die den Klassifizierungsbereich durchdringen, mindestens ein optisches Element und eine mit dem Prozessor kommunizierende interne Steuereinheit, wobei das elektrooptische Modul so konfiguriert ist, dass es mehrere Bilder des präadulten Insekts aus verschiedenen Winkeln erhält, und wobei die Lichtquellenbeleuchtung stroboskopisch oder gepulst ist.

6. Das System nach einem der Ansprüche 1 bis 5, das ferner einen mit der Steuereinheit kommunizierenden Sensor umfasst, wobei die Steuereinheit ferner so konfiguriert ist, dass sie die Anzahl der Insektenindividuen am Eingang des Strömungskanals auf der Grundlage der von dem genannten Sensor erfassten Daten steuert, um dadurch den Durchgang eines einzelnen präadulten Insekts in den Strömungskanal in einer bestimmten Zeit zu ermöglichen.

7. Das System nach einem der Ansprüche 1 bis 6, wobei der Prozessor so konfiguriert ist, dass er Funktionen ausführt, die ausgewählt sind aus: Empfangen der optischen Daten eines einzelnen präadulten Insekts, die von dem Sensor des elektrooptischen Moduls erfasst wurden, Verarbeiten der optischen Daten, Extrahieren eines Satzes von Parametern, die das Geschlecht des präadulten Insekts anzeigen, aus den optischen Daten, Klassifizieren des präadulten Insekts auf der Grundlage des Satzes von Parametern, die aus den optischen Daten extrahiert wurden, Bereitstellen von Anweisungen für die Steuereinheit auf der Grundlage der Klassifizierung des präadulten Insekts.

8. Das System nach Anspruch 7, wobei die Parameter, die das Geschlecht des präadulten Insekts anzeigen, aus der Gruppe ausgewählt sind, die aus der Gesamtgröße des präadulten Insekts, der Morphologie des präadulten Insekts, der Form, dem Segmentgrößenverhältnis, der IR (Infrarot)-Absorption, der Farbe, der Fluoreszenz, der Morphologie der Gonadenscheibe, der Morphologie des sekundären Geschlechtsorgans, der Gonadengröße, der Gonadenmorphologie, der Autofluoreszenz der Gonaden, der Größe der Hoden, der Größe der männlichen Nebendrüsen, Morphologie der Hoden, Morphologie der männlichen Nebendrüsen, Autofluoreszenz der Hoden, Autofluoreszenz der männlichen Nebendrüsen, Größe der sich entwickelnden männlichen und weiblichen primären oder sekundären Fortpflanzungsstrukturen, primitive Geschlechtsorgane, Morphologie der sich entwickelnden männlichen und weiblichen primären oder sekundären Fortpflanzungsstrukturen, Autofluoreszenz der sich entwickelnden männlichen und weiblichen primären oder sekundären Fortpflanzungsstrukturen und jeder Kombination davon besteht.

9. Das System nach einem der Ansprüche 1 bis 8, wobei das genannte System ferner einen Behälter umfasst, der in Fluidverbindung mit einem Strömungskanal zur Geschlechtertrennung von präadulten Insekten steht, wobei der genannte Behälter unsortierte präadulte Insekten enthält, die in einem flüssigen Medium suspendiert sind, und einen Auslass aufweist, der so konfiguriert ist, dass er den Durchgang jeweils eines einzelnen präadulten Insekts zum Strömungskanal ermöglicht, wobei der genannte Behälter ferner Druckmittel umfasst, wie beispielsweise Druckgas, Vakuum, Schwerkraft und/oder eine oder mehrere Pumpen, um einen Strom unsortierter präadulter Insekten zum Auslass des Behälters zum Strömungskanal zu treiben.

10. Ein computerimplementiertes Verfahren zur Geschlechtertrennung eines präadulten Insekts, das Folgendes umfasst:
a) Bereitstellung des Systems zur Geschlechtertrennung von präadulten Insekten nach einem der Ansprüche 1 bis 9;
b) Strömen der präadulten Insekten, die in dem flüssigen Medium suspendiert sind, durch den Einlass in den Strömungskanal in Richtung der Klassifizierungsregion;
c) Erfassung optischer Daten des einzelnen präadulten Insekts in der Klassifizierungsregion durch einen oder mehrere Sensoren des elektro-optischen Moduls;
d) Übermittlung der optischen Daten des einzelnen präadulten Insekts an den Prozessor;
e) Verarbeitung der optischen Daten und Klassifizierung des einzelnen präadulten Insekts auf der Grundlage einer Reihe von Parametern, die das Geschlecht des präadulten Insekts angeben, das aus den optischen Daten extrahiert wurde;
f) Bereitstellung von Anweisungen an die Steuereinheit auf der Grundlage der Klassifizierung des einzelnen präadulten Insekts; und;
g) Sortieren des präadulten Insekts gemäß den Anweisungen der Steuereinheit.

11. Das Verfahren nach Anspruch 10, wobei die präadulten Insekten in mindestens zwei Klassen eingeteilt werden, die aus einer Gruppe ausgewählt werden, die besteht aus:
männlich und nicht-männlich, oder weiblich und nicht-weiblich.

12. Das Verfahren nach einem der Ansprüche 10, 11, wobei das präadulte Insekt eine Larve, eine Puppe oder eine Nymphe ist.

13. Das Verfahren nach einem der Ansprüche 10 bis 12, wobei die präadulte Insektenart aus der Gruppe der Endopterygota ausgewählt ist, die aus folgenden Arten besteht: Coleoptera (Käfer), Diptera (Fliegen), Hymenoptera (Ameisen, Bienen, Sägefliegen und Wespen), Lepidoptera (Schmetterlinge und Motten), Mecoptera (Skorpionfliegen), Megaloptera (Erlenfliegen, Dobsonfliegen und Fischfliegen), Miomoptera, Neuroptera (Florfliegen, Antionen), Raphidioptera (Schlangenfliegen), Siphonaptera (Flöhe), Strepsiptera (Parasiten mit gedrehten Flügeln) und Trichoptera (Köcherfliegen), und wobei die präadulte Insektenart ausgewählt ist aus der Gruppe bestehend aus: a) Diptera-Arten, wie z. B. aus der Familie der Culicidae, z.B. Culex pipiens, Aedes Aegypti, Aedes albopictus, Anopheles gambiae; aus der Familie Drosophilidae, z.B. Drosophila melanogaster, Drosophila suzukir, aus der Familie Tephritidae, z.B. Bactrocera tryoni, Anastrepha fraterculus, Anastrepha ludens; aus der Familie Stratiomyidae, z.B. Hermetia illucens aus der Familie Syrphidae, z.B. Eristalis tenax b) Fepidoptera-Arten, wie aus der Familie Plutellidae, z.B. Plutella xylostella aus der Familie Pyralidae, z.B. Plodia interpunctella c) Coleoptera-Arten, wie aus der Familie Tenebrionidae, z.B. Tribolium castaneum.

14. Das Verfahren nach einem der Ansprüche 10 bis 13 umfasst ferner Schritte zur Verringerung oder Erhöhung der Temperatur des flüssigen Mediums, um die Zappelbewegungen der präadulten Insekten im Strömungskanal zu steuern.

15. Das Verfahren nach einem der Ansprüche 10 bis 14, wobei die Klassifizierung die Verwendung eines trainierten neuronalen Netzes umfasst.

## Revendications

1. Système de séparation par sexe d'insectes pré-adultes comprenant :
a) un canal fluidique (24) ayant un espace interne et une paroi externe, ledit canal fluidique comprenant une entrée et au moins une sortie (34, 36), chacune desdites une ou plusieurs sorties est associée à une catégorie de classification sélectionnée parmi le groupe constitué d'insecte mâle, d'insecte femelle et d'autre, dans lequel ledit canal est configuré pour permettre l'écoulement d'insectes pré-adultes (26) suspendus dans un milieu liquide ;
b) un processeur ; et
c) un contrôleur en communication avec ledit processeur ;
dans lequel ledit canal fluidique comprend une région de classification (28), et la région de classification comprend un module électro-optique en communication avec le processeur, ledit module électro-optique comprend au moins un capteur configuré pour acquérir des données optiques d'un insecte pré-adulte individuel tout en étant suspendu dans un milieu liquide et s'écoulant dans ledit canal fluidique, et pour transmettre lesdites données optiques au processeur, en outre dans lequel le processeur est configuré pour traiter les données optiques acquises, pour classifier ledit insecte pré-adulte en fonction des données optiques acquises, et pour instruire le contrôleur de trier ledit insecte pré-adulte en fonction de la classification dudit insecte pré-adulte.

2. Système selon la revendication 1, dans lequel ledit canal fluidique comprend plus d'une sortie, dans lequel chaque sortie est opérée indépendamment par le contrôleur ; la configuration de la sortie passe de « ouverte » à « fermée » et de « fermée » à « ouverte ».

3. Système selon la revendication 1 ou 2, dans lequel le canal fluidique comprend en outre une région de séparation (30), dans lequel ladite région de séparation est située entre la région de classification et la pluralité de sorties ; dans lequel ladite région de séparation comprend au moins un outil de guidage d'insecte configuré pour guider ledit insecte pré-adulte vers une sortie associée à la classification d'insecte, et dans lequel l'outil de guidage d'insecte est sélectionné parmi le groupe constitué d'une ou plusieurs vannes, d'un transducteur configuré pour générer des forces de rayonnement acoustique, d'électrodes configurées pour appliquer des forces électro-osmotiques au milieu liquide, d'électrodes configurées pour appliquer des forces diélectrophorétiques à l'insecte pré-adulte et de toute combinaison de ceux-ci.

4. Système selon l'une quelconque des revendications 1 à 3, dans lequel le canal fluidique comprend en outre une région de destruction ; dans lequel ladite région de destruction comprend au moins un outil de destruction d'insecte en communication avec le contrôleur ; et dans lequel ladite région de destruction d'insecte a une configuration « on » et « off » opérée par le contrôleur.

5. Système selon l'une quelconque des revendications 1 à 4, dans lequel le module électro-optique de la région de classification comprend au moins un élément parmi : une ou plusieurs sources lumineuses configurées pour éclairer la région de classification en émettant de la lumière avec un spectre et/ou des intensités prédéterminé(es), un ou plusieurs capteurs d'image, une ou plusieurs sources acoustiques configurées pour générer des ondes sonores qui passent à travers la région de classification, au moins un élément optique et un module de commande interne en communication avec le processeur, dans lequel le module électro-optique est configuré pour obtenir de multiples images de l'insecte pré-adulte de différents angles, et dans lequel l'éclairage par source lumineuse est stroboscopique ou pulsé.

6. Système selon l'une quelconque des revendications 1 à 5, comprenant en outre un capteur en communication avec le contrôleur, dans lequel le contrôleur est en outre configuré pour commander la quantité d'individus d'insecte à l'entrée vers le canal fluidique en fonction des données acquises par ledit capteur pour permettre de ce fait le passage d'un insecte pré-adulte unique à la fois vers le canal fluidique.

7. Système selon l'une quelconque des revendications 1 à 6, dans lequel le processeur est configuré pour réaliser des fonctions sélectionnées parmi la réception des données optiques d'un insecte pré-adulte individuel acquises par le capteur du module électro-optique, le traitement des données optiques, l'extraction d'un ensemble de paramètres indicateurs du sexe de l'insecte pré-adulte des données optiques, la classification de l'insecte pré-adulte en fonction de l'ensemble de paramètres extraits des données optiques, la fourniture d'instructions au contrôleur en fonction de la classification de l'insecte pré-adulte.

8. Système selon la revendication 7, dans lequel les paramètres indicateurs du sexe de l'insecte pré-adulte sont sélectionnés parmi le groupe constitué de la taille globale de l'insecte pré-adulte, de la morphologie de l'insecte pré-adulte, de la forme, du rapport de taille segmentaire, de l'absorption IR (infrarouge), de la couleur, de la fluorescence, de la morphologie de disque de gonade, de la morphologie d'organe sexuel secondaire, de la taille de gonade, de la morphologie de gonade, de l'autofluorescence de gonade, de la taille de testicules, de la taille de glandes accessoires mâles, de la morphologie de testicules, de la morphologie de glandes accessoires mâles, de l'autofluorescence de testicules, de l'autofluorescence de glandes accessoires mâles, de la taille des structures reproductives primaires ou secondaires mâles et femelles en développement, des organes sexuels primitifs, de la morphologie des structures reproductives primaires ou secondaires mâles et femelles en développement, de l'autofluorescence des structures reproductives primaires ou secondaires mâles et femelles en développement et de toute combinaison de ceux-ci.

9. Système selon l'une quelconque des revendications 1 à 8, dans lequel ledit système comprend en outre un récipient en connexion fluidique avec un canal fluidique pour la séparation par sexe d'insectes pré-adultes, ledit récipient contient des insectes pré-adultes non triés suspendus dans un milieu liquide, et ayant une sortie configurée pour permettre le passage d'un insecte pré-adulte unique à la fois vers le canal fluidique, ledit récipient comprend en outre un moyen de pressurisation, tel que du gaz pressurisé, du vide, des forces de gravité et/ou une ou plusieurs pompes pour entraîner un écoulement d'insectes pré-adultes non triés vers la sortie du récipient vers le canal fluidique.

10. Procédé mis en oeuvre par ordinateur de séparation par sexe d'insecte pré-adulte comprenant :
a) fournir le système de séparation par sexe d'insectes pré-adultes selon l'une quelconque des revendications 1 à 9 ;
b) faire ruisseler les insectes pré-adultes suspendus dans le milieu liquide à travers l'entrée dans le canal fluidique vers la région de classification ;
c) acquérir des données optiques de l'insecte pré-adulte individuel au niveau de la région de classification par un ou plusieurs capteurs du module électro-optique ;
d) transmettre les données optiques de l'insecte pré-adulte individuel au processeur ;
e) traiter les données optiques et classifier l'insecte pré-adulte individuel en fonction d'un ensemble de paramètres indicateurs du sexe de l'insecte pré-adulte extraits des données optiques ;
f) fournir des instructions au contrôleur en fonction de la classification de l'insecte pré-adulte individuel ; et
g) trier l'insecte pré-adulte en fonction des instructions reçues par le contrôleur.

11. Procédé selon la revendication 10, dans lequel les insectes pré-adultes sont classifiés en au moins deux classes sélectionnées parmi un groupe constitué de :
mâle et non mâle, ou femelle et non femelle.

12. Procédé selon l'une quelconque des revendications 10, 11, dans lequel l'insecte pré-adulte est une larve ou une pupe ou une nymphe.

13. Procédé selon l'une quelconque des revendications 10 à 12, dans lequel le type d'insecte pré-adulte est sélectionné parmi le groupe de Endopterygota constitué de : Coleoptera (scarabées), Diptera (mouches), Hymenoptera (fourmis, abeilles, mouches à scie et guêpes), Lepidoptera (papillons et papillons de nuit), Mecoptera (mouches-scorpions), Megaloptera (sialidés, Corydalinae et éphémères), Miomoptera, Neuroptera (chrysopes, fourmilions), Raphidioptera (raphidioptères), Siphonaptera (puces), Strepsiptera (parasites aux ailes tordues) et Trichoptera (porte-bois), et dans lequel l'espèce d'insecte pré-adulte est sélectionnée parmi le groupe constitué de : a) l'espèce Diptera, telle que provenant de la famille Culicidae, par exemple Culex pipiens, Aedes Aegypti, Aedes albopictus, Anopheles gambiae ; provenant de la famille Drosophilidae, par exemple Drosophila melanogaster, Drosophila suzukir, provenant de la famille Tephritidae, par exemple Bactrocera tryoni, Anastrepha fraterculus, Anastrepha ludens ; provenant de la famille Stratiomyidae, par exemple Hermetia illucens, provenant de la famille Syrphidae, par exemple Eristalis tenax, b) l'espèce Fepidoptera, telle que provenant de la famille Plutellidae, par exemple Plutella xylostella, provenant de la famille Pyralidae, par exemple Plodia interpunctella, c) l'espèce Coleoptera, telle que provenant de la famille Tenebrionidae, par exemple Tribolium castaneum.

14. Procédé selon l'une quelconque des revendications 10 à 13, comprend en outre les étapes de réduction ou d'élévation de la température du milieu liquide de manière à commander des mouvements de frétillement des insectes pré-adultes s'écoulant au sein du canal fluidique.

15. Procédé selon l'une quelconque des revendications 10 à 14, dans lequel ladite classification comprend utiliser un réseau neuronal appris.
